Europäisches Patentamt
European Patent Office
Office européen des brevets

(11) Publication number: 0 225 127 A2

# EUROPEAN PATENT APPLICATION

(21) Application number: 86309074.2

(22) Date of filing: 20.11.86

(51) Int. Cl.[4]: C 07 D 501/34

(30) Priority: 22.11.85 US 801109

(43) Date of publication of application: 10.06.87 Bulletin 87/24

(84) Designated Contracting States: AT BE CH DE ES FR GB GR IT LI LU NL SE

(71) Applicant: ELI LILLY AND COMPANY
Lilly Corporate Center
Indianapolis Indiana 46285 (US)

(72) Inventor: Webber, John Alan
7635 Cope Cod Circle
Indianapolis Indiana 46250 (US)

(74) Representative: Hudson, Christopher Mark et al
Erl Wood Manor
Windlesham Surrey GU20 6PH (GB)

Claims for the following Contracting States: AT + GR + ES.
A request for correction of the formula on page 2 (line 25) and in the claims 6 + 1 has been filed pursuant to Rule 88 EPC. A decision on the request will be taken during the proceedings before the Examining Division (Guidelines for Examination in the EPO, A-V, 2.2).

(54) **Esters of cefuroxime.**

(57) Dioxolenone esters of cefuroxime have oral absorptivity and give therapeutically useful blood levels. The esters have the formula

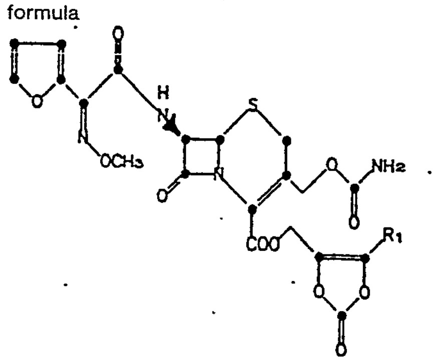

1

wherein $R_1$ is $C_1$ to $C_6$ alkyl or phenyl.

EP 0 225 127 A2

Bundesdruckerei Berlin

**Description**

Esters of Cefuroxime

This invention relates to orally acceptable esters of cefuroxime.

In an effort to control the ever-increasing price of health care, hospital patients are being released at an earlier date than ever before. The trend for early dismissal extends to patients suffering from infectious diseases. A highly desirable course of therapy for infectious disease patients is to treat them with the parenteral form of a broad spectrum antibiotic while they are hospitalized and, upon dismissal, continue the therapy with an oral form of the same antibiotic. The compounds of the instant invention facilitate such a course of therapy. The compounds are esters of the parenteral, broad-spectrum cephalosporin antibiotic cefuroxime. These particular esters are orally bioavailable forms of cefuroxime. Thus, the compounds of the instant invention provide the opportunity of continuing the nosocomial therapy of cefuroxime with the oral form of the compound once the patient is dismissed.

According to the present invention there are provided dioxo-lenone esters of Formula 1:

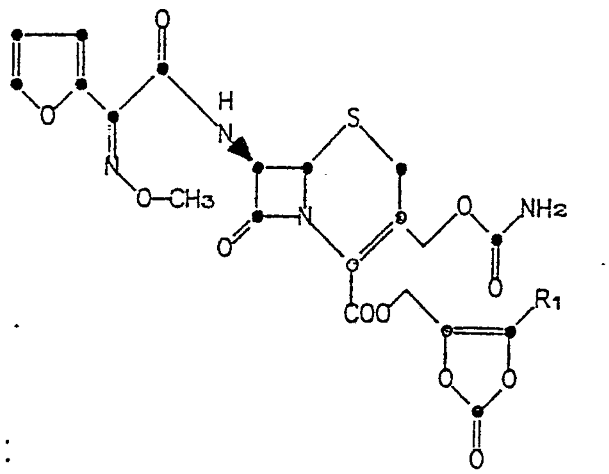

1

wherein $R_1$ is $C_1$ to $C_6$ alkyl or phenyl.

The oximino ether functionality in the above Formula is preferably in the "Z" configuration. Furthermore, the above Formula encompasses the corresponding hydrates and solvates. In the above Formula, the term "$C_1$ to $C_6$ alkyl" indicates groups such as methyl, ethyl, n-propyl, iso-propyl, n-butyl, iso-butyl, sec-butyl, N-pentyl, 3-methylbutyl, iso-pentyl, n-hexyl, 3-methylpentyl, 3,3-dimethylbutyl, and the like. Preferred dioxolenone esters of Formula 1 occur when $R_1$ is $C_1$ to $C_6$ alkyl, and especially so when $R_1$ is methyl.

The compounds of Formula 1 can be synthesized by esterifying cefuroxime (Formula 2)

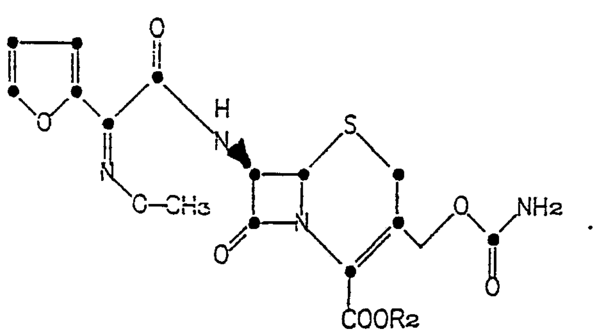

2

(wherein $R_2$ is hydrogen or a cation such as sodium, potassium or lithium ion) with a dioxolenone halide of Formula 3:

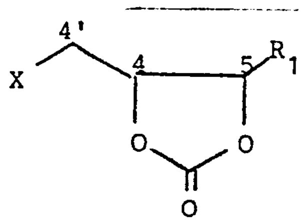

3

wherein $R_1$ is as defined above and X is chloro, bromo, or iodo. The esterification reaction is carried out in a substantially anhydrous aprotic organic solvent such as dimethylacetamide, dimethylformamide, dimethyl sulfoxide, acetone, ethyl acetate or mixtures of such solvents. Dimethylacetamide is the preferred solvent. The compounds of Formulas 2 and 3 are combined in approximately an equimolar amount, or the dioxolenone halide may be used in excess. If the free acid of cefuroxime (Formula 2, $R_2$ is hydrogen) is used as a precursor, a base is preferably added to the reaction mixture to convert the free acid to a carboxylate salt (Formula 2, $R_2$ is a cation). Examples of such bases include trialkylamines such as triethylamine and inorganic bicarbonates such as potassium bicarbonate and sodium bicarbonate. It is preferred that the carboxylate salt of cefuroxime be the precursor in the esterification reaction, and especially the sodium carboxylate salt. The esterification reaction can be carried out from about the freezing point of the solvent system to about 50° C, with about 0° C being the preferred temperature.

Further description of the esterification reaction is given below in the Experimental Section and in U.S. Patent No. 4,448,769, U.S. Patent No. 4,479,947, and U.S. Patent No. 4,389,408.

The dioxolenone halide of Formula 3 is preferably used as the bromide. Furthermore, the reagent can be isolated and purified before use in the esterification reaction or may be generated in situ immediately before use. The compounds of Formula 3 are generated by reacting the unsubstituted dioxoleneone (i.e., Formula 3 where X is hydrogen) with an allylic halogenating agent such as molecular chlorine, bromine, t-butyl hypoiodide, N-bromosuccinimide and N-chlorosuccimmide. The halogenation reaction can be run in the presence of a radical initiator such as 2,2'-azobis(iso-butyronitrile) and/or in the presence of ultraviolet light. The halogenation reaction is run in an inert aprotic organic solvent such as carbon tetrachloride, tetrahydrofuran, 1,2-dimethoxyethane, benzene, tetrachloroethylene, and the like. Carbon tetrachloride is the preferred solvent. The reaction can be carried out from about room temperature to about the refluxing temperature of the solvent system, with the refluxing temperature of carbon tetrachloride being the preferred temperature.

Further details on the preparation of the dioxo-lenone halides of Formula 3 are given below in the Experimental Section and in U.S. Patent No. 4,342,693.

The preparation of the compound of Formula 2 (cefuroxime) is set forth in U.S Patent No. 3,974,153.

Upon oral administration, dissolution, enteric absorption, hydrolysis, and entry of cefuroxime into the blood, the present dioxolenone esters become antibacterially effective against the same range of gram-positive and gram-negative pathogens as cefuroxime. Cefuroxime is poorly absorbed through the intestine. The presence of the dioxolenone ester on the cefuroxime molecule increases oral absorption and blood levels of cefuroxime over the levels obtained after the oral administration of unesterified cefuroxime. The enhancement of oral absorption exhibited by the dioxolenone ester-forming moiety on cefuroxime is evinced by the 5-methyldioxolenone ester compound's oral bioavailability in test animals such as mice and dogs.

Table 1 below sets forth oral absorption test data for mice. The values shown are averaged values for three animals at each test point. The test animals were dosed with 20 milligrams/kilogram of the 5-methyldioxolenone ester of cefuroxime (which equates to 15 milligrams/kilogram of cefuroxime free acid).

Table 1

Mouse Plasma Levels (μg/ml)

| Time from dose (minutes) | | | | | | | % of dose in urine |
|---|---|---|---|---|---|---|---|
| 5 | 20 | 40 | 60 | 80 | 100 | 120 | |
| 2.2 | 5.4 | 3.7 | 1.8 | <1.7 | <1.7 | <1.7 | 25.1% |

The cefuroxime dioxolenone ester compounds of this invention are useful for the therapeutic and prophylactic treatment of infections in warm-blooded animals caused by gram-positive and gram-negative

bacteria. The antibiotic compounds are administered orally to the infected animal.

A further aspect of this invention is a pharmaceutical formulation suitable for oral administration, comprising a suitable vehicle and an antibacterially effective amount of a compound of Formula 1. These compositions are useful for the prevention and control of gram-positive and gram-negative bacterial infections. The pharmaceutical formulations can be in the form of capsules, tablets, lozenges, liquid suspensions and the like. The dioxolenone ester oompounds may be in either crystalline or amorphous form when used in conjunction with the instant formulations.

With regard to the formulations (such as tablets and capsules), the term "suitable vehicle" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; disintegrators such as micro-crystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical formulations of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "suitable vehicle" means conventional additives such as suspending agents, for example, sorbitol, syrup, methyl cellulose, glucose/sugar syrup, gelatin, hydroxyethylcellulose, carboxymethyl cellulose, aluminium stearate gel, or hydrogenated edible oils, for example, almond oil, fractionated coconut oil, oily esters, propylene glycol or ethyl alcohol; and preservatives such as methyl or propyl p-hydroxybenzoates or sorbic acid.

One preferred pharmaceutical formulation occurs when $R_1$ in the above Formula is methyl. Other preferred pharmaceutical formulation are capsules and tablets in unit dosage form. The unit dosage may be from about 100 milligrams to about 1 gram. Unit dosages may be obtained by filling capsules with the desired dosage. Capsules containing 125 milligrams, 250 milligrams, and 500 milligrams of the dioxolenone ester of cefuroxime compound are preferred. Unit dosages in tablet form can contain 125 milligrams, 250 milligrams, or 500 milligrams of the dioxolenone ester compound per tablet. More preferred pharmaceutical formulations when the formulation is in tablet and capsule form occurs when $R_1$ in the above Formula 1 is methyl.

An "antibacterially effective amount" of the compounds of Formula I is from approximately 2.5 mg to about 50 mg of compound per kilogram of body weight. This amount generally totals from about 0.5 gram to about 12 grams per day for an adult human.

A further aspect of this invention is a method for treating or controlling infectious diseases caused by gram-positive organisms in warm-blooded animals. This method comprises administering to the animal an antibacterially effective amount of the compounds of Formula 1. A typical daily dose for an adult human in this method is from about 0.5 gram to about 12 grams.

In practicing this method, the compounds of Formula 1 can be administered in a single daily dose or in multiple doses per day. The treatment regime may require adminis-tration over extended periods of time, for example, for several days or for from two to three weeks. The amount administered per dose or the total amount administered will depend on such factors as the nature and severity of the infection, the age and general health of the patient, and the tolerance to the compounds of Formula 1 of both the patient and the microorganism or microorganisms involved in the infection.

The following Examples are provided to further illustrate the invention. It is not intended that the invention be limited in scope by reason of the following Examples.

In the following Examples, the terms nuclear magnetic resonance spectrum, field desorption mass spectrum, infrared spectrum, ultraviolet spectrum, and high performance liquid chromatography are abbreviated n.m.r., f.d.m.s., i.r., u.v., and HPLC, respectively. In addition, the adsorption maxima listed for the i.r. spectrum are only those of interest and not all of the maxima observed.

The abbreviations "AIBN", "DMAC", "mmol" stand for 2,2′-azobis[2-isobutyronitrile], dimethylacetamide, and millimole, respectively.

In conjunction with the n.m.r. spectrum, the following abbreviations are used: "s" is singlet, "d" is doublet, "q" is quartet, and "m" is multiplet. "J" indicates the coupling constant in Hertz. "DMSO-$d_6$" is dimethyl sulfoxide where all protons have been replaced with deuterium.

The n.m.r. spectrum was obtained on a General Electric Model QE-300 instrument. The chemical shifts are expressed in δ values (parts per million downfield from tetramethylsilane). The infrared spectrum was obtained on a Nicolet Model 10-MX instrument. The ultraviolet spectrum was obtained on a Cary Model 118 instrument.

Example 1

(5-Methyl-2-oxo-1,3-dioxolen-4-meth-4'-yl)
(7R)-7-[(2-(fur-2-yl)-2-Z-methoximino)acetamido]-3-(aminocarbonyloxymethyl)-3-cephem-4-carboxylate

A. Preparation of 5-Methyl-2-oxo-1,3-dioxolen-4-methyl-4'-bromide

4,5-Dimethyl-2-oxo-1,3-dioxolene (3.4 g, 30 mmol), N-bromosuccinimide (5.34 g), and AIBN (trace) was added to carbon tetrachloride. The resultant solution was refluxed for approximately 0.5 hr, cooled, and concentrated in vacuo to one-half the original volume. The concentrate was filtered then further concentrated to yield an oil of 5-methyl-2-oxo-1-3-dioxolen-4-methyl-4'-bromide.

B. Esterification of sodium
(7R)-7-[(2-(fur-2-yl)-2-Z-methoximino)acetamido]-3-(aminocarbonyloxymethyl)-3-cephem-4-carboxylate.

Sodium (7R)-7-[(2-(fur-2-yl)-2-Z-methoximino)-acetamido]-3-(aminocarbonyloxymethyl)-3-cephem-4-carboxylate (9.8 g) was dissolved in DMAC (50 ml). Molecular sieves were added to the solution and the resultant mixture was cooled in an ice bath. The oil from Procedure A above was dissolved in DMAC (50 ml) and added dropwise over a 70 minute period to the cooled mixture. The resultant mixture was stirred for approximately one hour, filtered, and the residue washed with DMAC. The mother liquor was dropped into water (700 ml) resulting in a precipitate. The precipitate was collected by filtration and dried in vacuo to give 9.1 g of (5-methyl-2-oxo-1,3-dioxolen-4-meth-4'-yl) (7R)-7-[(2-(fur-2-yl)-2-Z-methoximino)acetamido]-3-(aminocarbonyloxymethyl)-3-cephem-4-carboxylate: n.m.r. (300 MHz, DMSO-$d_6$) δ 2.2 (s, 5-methyl group of dioxolenone group), 2.5 (m, solvent), 2.8 (s), 2.95 (s), 3.35 (s, water), 3.6 (q, $C_2$ methylene protons), 3.9 (s, methyl protons of methoxime group), 4.7 (q, $C_3'$ methylene protons), 5.15 (d, $C_6$ proton), 5.25 (m, 4-methylene protons of the dioxolenone group), 5.85 (q, $C_7$ proton), 6.65 (m, furyl group protons), 7.95 (s, carbamato nitrogen protons), 9.8 (d, $C_7$ amido nitrogen proton); i.r. (Nujol mull): 1821, 1785, 1734 $cm^{-1}$; u.v. (ethanol): $\lambda_{max} = 276$ ($\varepsilon_{max} = 19{,}554$).

Anal. Calcd for $C_{21}H_{20}N_4O_{11}S$: C, 47.02; H, 3.76; N, 10.44.
Found: C, 46.78; H, 3.98; N, 10.22.

Example 2

(5-Methyl-2-oxo-1,3-dioxolen-4-methyl-4'-yl) (7R)-7-[(2-(fur-2-yl)-2-Z-methoximino)acetamido]-3-(aminocarbonyloxymethyl)-3-cephem-4-carboxylate, 250 mg, is formulated into tablet form with starch and magnesium stearate.

Example 3

(5-Methyl-2-oxo-1,3-dioxolen-4-meth-4'-yl) (7R)-7-[(2-(fur-2-yl)-2-Z-methoximino)acetamido]-3-(aminocarbonyloxymethyl)-3-cephem-4-carboxylate as a dry crystalline powder is filled in 125 mg portions into gelatin capsules to provide 125 mg unit dosages.

Example 4

(5-Methyl-2-oxo-1,3-dioxolen-4-meth-4'-yl) (7R)-7-[(2-(fur-2-yl)-2-Z-methoximino)acetamido]-3-(aminocarbonyloxymethyl)-3-cephem-4-carboxylate is filled in 500 mg portions into gelatin capsules to provide 500 mg unit dosages.

Example 5

(5-Methyl-2-oxo-1,3-dioxolen-4-meth-4'-yl) (7R)-7-[(2-(fur-2-yl)-2-Z-methoximino)acetamido]-3-(aminocarbonyloxymethyl)-3-cephem-4-carboxylate as a dry powder is filled in 250 mg portions into gelatin capsules to form 250 mg unit dosages.

## Claims

1. A compound of the formula 1:

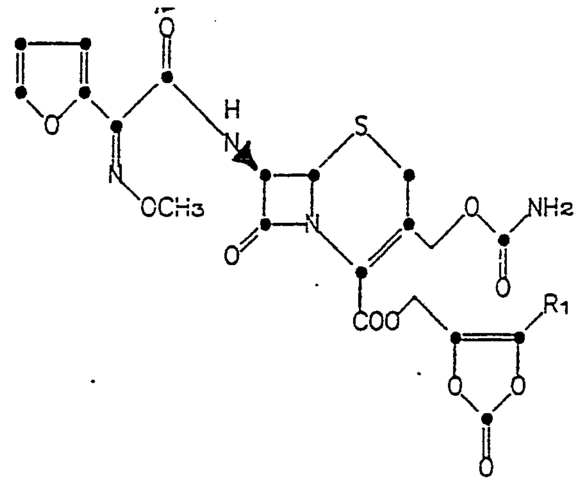

1

wherein $R_1$ $C_1$ to $C_6$ alkyl or phenyl.

2. A compound of claim 1, wherein $R_1$ is $C_1$ to $C_6$ alkyl.

3. A compound of claim 2, wherein $R_1$ is methyl.

4. A pharmaceutical formulation suitable for oral administration, comprising a compound of formula 1, as claimed in any one of claims 1 to 3.

5. A compound of formula 1, as claimed in any one of claims 1 to 3 for use in the therapy of bacterial infections.

6. A process for preparing a compound of formula 1, as claimed in any one of claims 1 to 3, which comprises esterifying

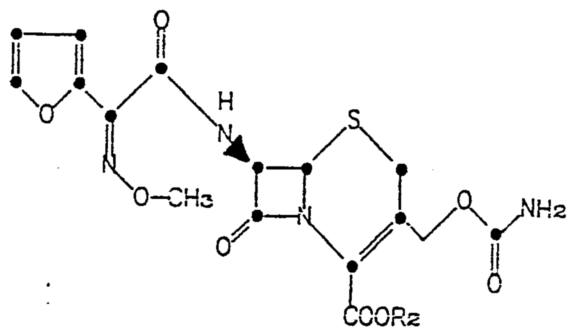

2,

wherein $R_2$ is hydrogen or a cation, with a dioxolenone halide of

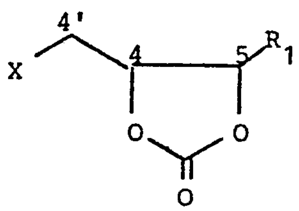

3

wherein X is chloro, bromo, or iodo.

Claims for the following Contracting States : AT; GR and ES

1. A process for preparing a compound of the formula 1:

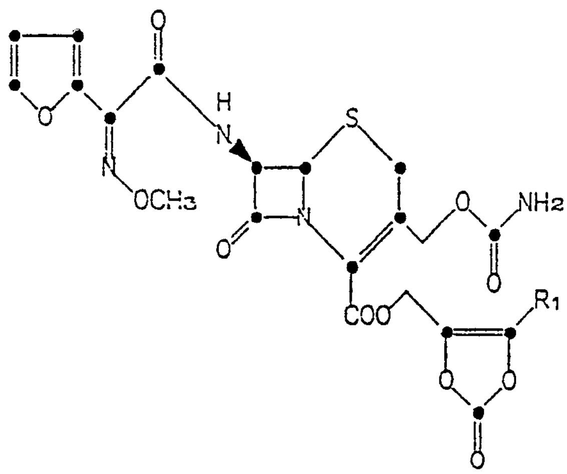

1

wherein $R_1$ is $C_1$ to $C_6$ alkyl or phenyl, which comprises esterifying

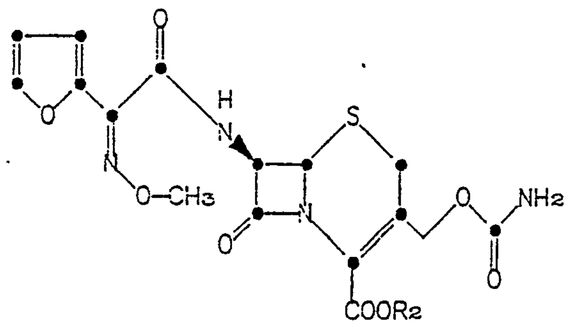

2,

wherein $R_2$ is hydrogen or a cation, with a dioxolenone halide of

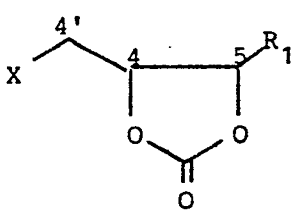

3

wherein X is chloro, bromo, or iodo.

2. A process according to claim 1, wherein $R_1$ is methyl.